# EUROPÄISCHE PATENTSCHRIFT

(11) **EP 0 547 544 B1**
(45) Veröffentlichungstag und Bekanntmachung des Hinweises auf die Patenterteilung: **12.03.1997**
(21) Anmeldenummer: 92121255.1
(22) Anmeldetag: 14.12.1992
(51) Int. Cl.: C07K 14/62, C07K 1/20

(54) **Verfahren zur Gewinnung von insulinhaltigen Lösungen**
Process for the preparation of insulin-containing solutions
Procédé de préparation de solutions contenant de l'insuline

(30) Priorität: 18.12.1991 DE 4141794; 20.06.1992 DE 4220293
(43) Veröffentlichungstag der Anmeldung: 23.06.1993
(73) Patentinhaber: HOECHST AKTIENGESELLSCHAFT, 65926 Frankfurt am Main (DE)
(72) Erfinder: Dickhardt, Rainer, Dr., W-6233 Kelkheim/Ts. (DE); Unger, Bernhard, Dr., W-6272 Niedernhausen-Oberjosbach (DE); Gräfe, Claudia, W-6240 Königstein/Ts. (DE)

(56) Entgegenhaltungen:
- EP-A- 0 474 213
- WO-A-89/01485
- JOURNAL OF CHROMATOGRAPHY Bd. 265, Nr. 2 , 1983 , AMSTERDAM NL Seiten 301 - 309 B.S. WELINDER ET AL. 'Separation, Isolation and Characterization of the four Monoiodinated Insulin Tracers using Reversed-Phase High-Performance Liquid Chromatography'
- JOURNAL OF CHROMATOGRAPHY Bd. 461 , 1989 , AMSTERDAM NL Seiten 45 - 61 E.P. KROEFF ET AL. 'Production Scale Purification of Biosynthetic Human Insulin by Reversed-Phase High-Performance Liquid Chromatography'
- W.S. Hancock and R.L Prestidge 'Biochemical Applications of Preparative Liquid Chromatography' in Preparative liquid chromatography ed. by
- J. OF CHROMATOGRAPHY, Bd. 265, Nr. 2, 1983, Amsterdam, NL, Seiten 301-309, B.S. WELINDER et al.
- J. OF CHROMATOGRAPHY, Bd. 461, 1989, Amsterdam, NL, Seiten 45- 61, E.P. KROEFF et al.
- J. OF CHROMATOGRAPHY LIBRARY, Bd. 38, Kap. 6, 1987, Herausgeber B.A. Bidlingmeyer, Seiten 203-231, W.S. HANCOCK et al.

## Beschreibung

Aus analytischen Trennverfahren ist die Reinigung von Insulinen oder Insulinderivaten an lipophil modifizierten (reversed phase) Kieselgelen bekannt und wird seit vielen Jahren in der Hochdruckflüssigkeitschromatographie (HPLC) erfolgreich angewendet (WS Welinder et al., J. Chrom., 361, (1986)357 - 367). Im analytischen Maßstab werden Proteinmengen im µg-Bereich auf eine mit modifiziertem Kieselgel gefüllte Säule aus Stahl, Glas oder Kunststoff aufgetragen und anschließend durch ein strömendes Flüssigkeitsgemisch (zumeist saure, wäßrige Pufferlösungen mit konstanter oder variabler organischer Lösemittelkonzentration) eluiert. Die Proteinbeladung beträgt hierbei weit weniger als 30 µg/ml Säulenvolumen.

Insuline aus vorherigen chemischen Umwandlungen wie beispielsweise aus stark sauren Esterspaltungen oder enzymatischen (Trans-)Peptidierungsprozessen und aus Kristallisationsreinigungen enthalten zumeist Begleitstoffe mit ähnlichen Eigenschaften. Sie lassen sich durch die Wahl bestimmter pH-Werte über Ionenaustauschchromatographie reinigen, wenn genügend elektrische Ladungsunterschiede vorhanden sind (US 4 129 560). Der Nachteil dieser Methode liegt im Verdünnungseffekt und damit Verlust an Wertstoffen in den Überständen bei der Aufarbeitung der Fällungen, in der relativ langen Zykluszeit oder darin, daß die Gesamtwiederfindung und damit die Ausbeute geringer ist.

Insulinzubereitungen mit Depoteigenschaften enthalten häufig Protamine, die die Wirkdauer von Insulinen verlängern. Protamine sind argininreiche Proteine, die aus Rogen stammen. Qualitätsuntersuchungen von Depotinsulinzubereitungen zeigten immer wieder einen unerklärlichen Verlust der Depoteigenschaften nach längerer Lagerung der Zubereitung. Solche Insulinzubereitungen sind für den Patienten völlig ungeeignet, weil es damit leicht zu einer Insulinüberdosierung kommt, die unter Umständen über einen hypoglykämischen Schock zum Tod des Patienten führen kann.

Genaue Untersuchungen dieser Insulinzubereitungen zeigten, daß der Verlust der Depoteigenschaften durch einen langsamen Abbau der Protamine durch Spuren von Proteasen verursacht wird. Ferner zeigte es sich, daß die Hauptmenge an Proteasen in diesen Zubereitungen aus der enzymatischen Transpeptidierung von Schweineinsulin zu Humaninsulin-Ester/Ether oder aus proteolytischen Spaltprozessen von insulinähnlichen Vorprodukten stammt. Ein Großteil der Proteasen, beispielsweise Trypsin, wird zwar durch die gängigen Chromatographieverfahren abgetrennt, trotzdem erreichen offensichtlich Restmengen an Proteasen die Insulinzubereitungen und führen dort zum Verlust der Depoteigenschaften.

Bei der gentechnischen Herstellung von Insulin wird als Nebenprodukt auch das an der Position A9 befindliche Serin des Insulins von dem Mikroorganismus acetyliert, so daß ein an der Hydroxylgruppe von Serin acetyliertes Insulinderivat entsteht. Dieses Insulinderivat wird bisher nur bei der gentechnischen Herstellung von Insulin mit Escherichia coli beobachtet. Die vollständige Abtrennung dieses acetylierten Insulinderivats von Humaninsulin gelingt bisher nur auf Kosten einer niedrigen Ausbeute an Humaninsulin, wenn man das in der Europäischen Patentanmeldung mit der Veröffentlichungs Nr. 0 474 213 beschriebene Verfahren verwendet. Ferner zeigt sich bei diesem Verfahren, daß keine Auftrennung mehr durchführbar ist, wenn die Konzentration von acetyliertem Insulin 5 % überschreitet. Ferner ist die annähernd proteasefreie Gewinnung von Insulin durch dieses Verfahren nicht erreichbar.

Es wurde nun ein Verfahren gefunden zur Gewinnung von Insulin, das annähernd frei ist von Proteasen und/oder an Position A9-acetyliertem Insulin durch Chromatographie in wäßrigen, gepufferten Elutionsmitteln, die mit Wasser mischbare, organische Lösungsmittel enthalten, an lipophil modifiziertem Kieselgel, das dadurch gekennzeichnet ist, daß man
1A) das Gemisch aus Proteasen und/oder an Position A9-acetyliertem Insulin und Insulin in einem Puffer löst,
1B) die Elution mit einem gepufferten Elutionsmittel, das Zwitterionen enthält und gegebenenfalls auf einen pH-Wert in der Nähe des isoelektrischen Punktes des zu reinigenden Insulins eingestellt worden ist, durchführt
die gewonnenen Insulinfraktionen gegebenenfalls einengt und anschließend die gewonnenen Insulinfraktionen
2A) in einem Auflösepuffer, der Aceton oder Acetonitril enthält, löst,
2B) die erhaltene Lösung auf die Säule aufträgt,
2C) die Säule mit dem Auflösepuffer nachspült und
2D) das zu reinigende Insulin mit einem gepufferten Elutionsmittel, das Zwitterionen enthält und gegebenenfalls auf einen pH-Wert in der Nähe des isoelektrischen Punktes des zu reinigenden Insulins eingestellt worden ist, eluiert.

Überraschenderweise bewirkt die Anwesenheit von Aceton oder Acetonitril im Auflösepuffer des Verfahrensschritts 2A die deutlich verbesserte Abtrennung der Proteasen und der an Position A9-acetylierten Insuline von Insulin, so daß die beschriebenen Probleme mit Insulinformulierungen, die Protamine enthalten, nicht mehr auftreten.

Die zu reinigenden Gemische aus Proteasen und/oder an Position A9-acetyliertem Insulin und Insulin können aus einer Vielzahl enzymatischer Prozesse stammen, beispielsweise proteolytische Abspaltung von Prä- und/oder Prosequenzen von Präproinsulinen, Abspaltung von C- oder N-terminalen Aminosäuren des Insulins oder Umamidierungen von Schweineinsulin zu Humaninsulin oder Humaninsulinderivaten. Ferner kann das in den zu reinigenden Gemischen enthaltende an Position A9-acetylierte Insulin aus einer Vielzahl gentechnisch exprimierter Insulin-Konstrukte stammen.

Die im Verfahren abtrennbaren Proteasen sind beispielsweise Trypsin, Lysylendopeptidase, Carboxypeptidase A, Clostripain oder Achromobacter-Protease. Bevorzugt ist Trypsin.

In der vorliegenden Anmeldung wird unter dem Begriff Insulin die folgenden Verbindungen verstanden. Insuline die tierischen oder menschlichen Ursprungs sind, z. B. Humaninsulin oder Schweineinsulin, Insulinvorläufer, wie z. B. Proinsuline oder Präproinsuline, oder rekombinante Insuline oder Insulinderivate, die von genetisch modifizierten Mikroorganismen exprimiert werden. Ferner können auch Insulinderivate eingesetzt werden, die beispielsweise durch chemische oder enzymatische Derivatisierung hergestellt wurden, z. B. Des-Phe-B1-Insulin, Insulin-β-Ketensulfonat, Diarginininsulin (B31, B32), Monoarginininsulin, Diphenylalanininsulin (B31, B32) (US 4 601 852), oder Gly^{A21}-Arg^{B31}-Arg^{B32}-Humaninsulin (EP 368 187).

Im erfindungsgemäßen Verfahren werden bevorzugt Insuline der Formel I eingesetzt 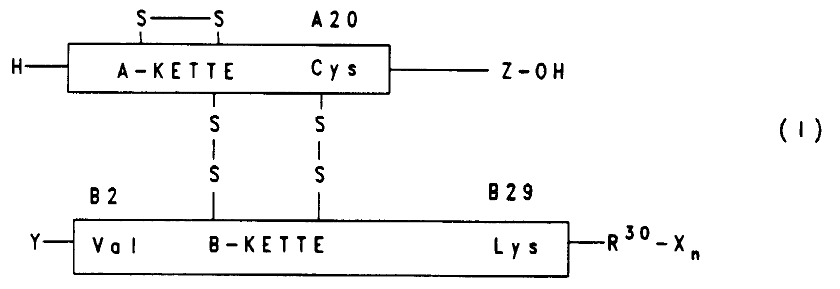 in welcher
- R³⁰: für den Rest einer genetisch kodierbaren L-Aminosäure,
- X: für eine Hydroxygruppe, eine physiologisch unbedenkliche organische Gruppe basischen Charakters mit bis zu 50 C-Atomen, eine genetisch kodierbare L-Aminosäure und deren gegebenenfalls vorhandene endständige Carboxylfunktion frei, als Esterfunktion, als Amidfunktion, als Lacton oder zu CH₂OH reduziert vorliegen kann,
- n: für eine ganze Zahl von 0 bis 10,
- Y: für Wasserstoff oder L-Phenylalanin, und
- Z: für eine genetisch kodierbare L-Aminosäure steht
und die A- und B-Kette die Sequenzen tierischen oder menschlichen Insulins haben.

Bevorzugt werden Insuline der Formel I, in welcher
- R³⁰: für L-Alanin oder L-Threonin und
- X: für eine oder mehrere L-Aminosäuren aus der Gruppe L-Arginin, L-Lysin oder L-Phenylalanin und
- Z: für L-Glycin, L-Alanin, L-Serin, L-Threonin, L-Asparaginsäure oder L-Glutaminsäure steht, und A1 bis A20 oder B2 bis B29 für die Aminosäuresequenz von Human-, Schweine- oder Rinderinsulin steht, eingesetzt.

Die Aminosäuresequenz A1 bis A20 von Humaninsulin ist:

Gly Ile Val Glu Gln Cys Cys Thr Ser Ile Cys Ser
Leu Tyr Gln Leu Glu Asn Tyr Cys (SEQ ID NO: 1)

Die Aminosäuresequenz B1 bis B29 von Humaninsulin ist:

Phe Val Asn Gln His Leu Cys Gly Ser His Leu
Val Glu Ala Leu Tyr Leu Val Cys Gly Glu Arg
Gly Phe Phe Tyr Thr Pro Lys (SEQ ID NO: 2)

Ferner wurde gefunden, daß sich die Verfahrensschritte 2A bis 2D des obengenannten Chromatographieverfahrens alleine ohne die Verfahrensschritte 1A und 1B hervorragend zur Gewinnung von Insulin aus Insulingemischen eignet, enthaltend Insulin, an der Position A9-acetyliertes Insulin und Proteasen, sowie Insulingemische, enthaltend Insulin und an Position A9-acetyliertes Insulin.

Die im erfindungsgemäßen Verfahren abtrennbaren an Position A9-acetylierten Insuline sind beispielsweise das an der Position A9-acetylierte Humaninsulin oder A9-acetylierte Insulin der Formel I, wobei R³⁰, X, n, Y und Z die obengenannten Definitionen haben.

Bevorzugt werden Insulinderivate der Formel I von den an Position A9-acetyliertem Insulinen der Formel I abgetrennt. Insbesondere erfolgt eine Abtrennung von an A9-acetyliertem Humaninsulin von Humaninsulin.

Insulin und Insulinderivate können sowohl in verhältnismäßig unreinem Zustand als auch in vorgereinigter Form (z. B. durch Gelchromatographie) eingesetzt werden. Insulin ist nach mehrfacher Kristallisation und auch nach Gelchromatographie noch mit insulinähnlichen Begleitstoffen sehr ähnlichen Molekulargewichts verunreinigt, die sich bei passend gewähltem pH in ihrem Ladungszustand untereinander und vom Insulin unterscheiden, aber mit Insulin Komplexe bilden (US 4 129 560). Beispiele solcher Substanzen sind Desamidoinsuline, Arginin- und Diarginininsulin und Insulinethylester.

Unter lipophil modifiziertem Kieselgel wird ein Kieselgel verstanden, auf das eine hydrophobe Matrix aufgebracht wurde. Beispiele für eine hydrophobe Matrix sind Alkane mit einer Kettenlänge von 3 bis 20 Kohlenstoffatomen, insbesondere 8 bis 18 Kohlenstoffatomen. Ferner kann die Korngröße in einem weiten Bereich schwanken, beispielsweise von 5 bis 60 µm, insbesondere von 10 bis 50 µm. Die Porenweite kann auch in einem weiten Bereich schwanken, günstige Porenweiten liegen bei 50 bis 300 Å, insbesondere 100 bis 200 Å. Beispiele für liphophil modifizierte Kieselgelmaterialien sind:
- ®Nucleosil, Firma Macherey & Nagel GmbH + Co.KG, Düren, Deutschland sphärische und nicht sphärische Materialien verschiedener Korngröße bis zu 45 µm, 100 Å Porenweite, C8- bzw. C18-modifiziert.
- ®LiChroprep, Firma E.Merck, Darmstadt, Deutschland nicht sphärische und sphärische Materialien verschiedener Korngrößen bis 40 µm, 60 - 250 Å Porenweite, C8- bzw. C18-modifiziert;
- ®LiChrospher Select B, Firma E.Merck, Darmstadt, Deutschland sphärisches Material bis 25 µm Korngröße, C8-modifiziert;
- ®Waters Prep, Firma Millipore GmbH, Eschborn, Deutschland C18-modifiziert, 50 - 105 µm
   nichtsphärisch, 100 Å Porenweite;
- ®Zorbax Pro10, Firma DuPont de Nemours (Deutschland) GmbH, Bad Homburg, Deutschland
   C8-modifiziert, 10 µm, sphärisch, 100 Å Porenweite;
- ®Kromasil, Firma EKA Nobel, Nobel Industries, Schweden C4-, C8- und C18-modifiziert, bis 20 µm, sphärisch, 100, 150 oder 200 Å Porenweite.

Zwitterionen sind Verbindungen, die Protonen aufnehmen und auch abspalten können, d. h. in saurer Lösung Kationen und in alkalischer Lösung Anionen bilden, wie beispielsweise α-Aminosäuren, Betain oder Betainderivate. Bevorzugte Zwitterionen sind Glycin, Glutamin oder Betain (N-Trimethyl-glycin). Besonders bevorzugt ist Glycin.

Der isoelektrische Punkt (IEP) eines Insulins oder Insulinderivates ist derjenige pH-Wert, in der die Summe der kationischen Ladungen und anionischen Ladungen des gelösten Insulins gleich Null ist. Beispielsweise liegt der IEP von Schweineinsulin im Bereich von pH 5,3 bis 5,4. Mit dem Begriff "in der Nähe des isoelektrischen Punkts" sind insbesondere pH-Werte gemeint, die um ca. 1 pH-Einheit über oder unter dem IEP des zu reinigenden Insulins liegen. Besonders bevorzugt sind pH-Werte, die bis zu 0,5 pH-Einheiten über oder unter dem IEP liegen.

Unter dem Begriff "annähernd proteasefreies Insulin" werden Mischungen von Insulin mit Proteasen verstanden bei denen die Proteaseaktivität 0 bis 4 mAU/min, bevorzugt 0,01 bis 1 mAU/min, insbesondere 0,02 bis 0,08 mAU/min, beträgt.

Die Proteaseaktivität wird durch Messung der Kinetik der Abspaltung eines Chromophors (4-Nitroanilid) von dem Substrat Carbobenzoxy-Val-Gly-Arg-4-nitroanilid-acetat (Chromozym-Try; Best.-Nr. 378488 Fa. Boehringer Mannheim) bestimmt. Die Abspaltungsreaktion wird spektralphotometrisch über einen Zeitraum von 60 min bei einer Wellenlänge von 405 nm gemessen. Die Zunahme der gemessenen Absorption ist dabei direkt proportional zur tryptischen Aktivität in dem Produkt. Die Steigung der Absorptionsgeraden hat als Einheit: milli absorption units pro Minute (mAU/min.).

Unter dem Begriff "Insulin, das annähernd frei ist von an der Position A9-acetyliertem Insulin" sind Insulinpräparationen gemeint, die einen Gehalt an A9-acetyliertem Insulin haben, der geringer ist als 0,5 %, bevorzugt weniger als 0,2 %, insbesondere weniger als 0,1 %.

Die Elutionsmittel enthalten eine Puffersubstanz, die den pH-Wert des Elutionsmittels konstant halten. Geeignete Puffersubstanzen sind in der Literatur bekannt, beispielsweise Phosphate, Alkali- oder Erdalkalimetallsalze wie Natriumcitrat oder Kaliumacetat, Ammoniumcitrat, -acetat, -sulfat oder -chlorid. Ferner enthalten die Elutionsmittel mit Wasser mischbare organische Lösungsmittel wie beispielsweise Alkohole, Ketone, Methylacetat, Dioxan oder Acetonitril. Bevorzugt werden Alkohole wie n- oder iso-Propanol, Methanol, Ethanol oder Methylacetat.

Für den ersten Chromatographieschritt (Verfahrensschritte 1A und 1B) beträgt die Konzentration der mit Wasser mischbaren organischen Lösungsmittel 1 bis 70 %, bevorzugt 10 bis 50 %, besonders bevorzugt 10 bis 35 %. Die Konzentration der Puffersubstanz beträgt etwa 1 mMol/l bis 140 mMol/l, bezogen auf Wasser als Lösungsmittel, bevorzugt 2 mMol/l bis 120 mMol/l. Die Konzentration der Zwitterionen kann in einem weiten Bereich schwanken. Vorteilhafte Mengen sind 1 mMol/l bis 140 mMol/l, bezogen auf Wasser als Lösungsmittel, bevorzugt 10 mMol/l bis 110 mMol/l.

Die Temperatur während der Chromatographie beträgt 0 °C bis 50 °C, vorzugsweise 15 bis 30 °C, besonders bevorzugt 15 bis 20 °C. Der Betriebsdruck während der Chromatographie ist weitgehend konstant. Die Chromatographie kann mit unterschiedlichem Druck durchgeführt werden, z. B. kann die Chromatographie bei einem Druck von 5 bis 400 bar durchgeführt werden, insbesondere bei 20 bis 100 bar.

Die Beladung der Säulen, Chromatographie und Elution der Insuline und Insulinderivate erfolgt nach bekannten, üblichen, technischen Methoden. Die Beladung der Säule mit der zu reinigenden Insulinlösung erfolgt bevorzugt mit wäßrig-alkoholischer oder rein wäßriger Pufferlösung. Die Insulinlösung hat einen Proteinanteil von etwa 1 bis 10 %, bevorzugt 3 %.

Die Elution der Insuline erfolgt mit dem erfindungsgemäßen Verfahren bei konstanter Konzentration der Puffersubstanzen (isokratisch) oder durch Veränderung des mit Wasser mischbaren organischen Lösungsmittelanteils im Puffer. Die Veränderung des organischen Lösungsmittelanteils erfolgt in der Weise, daß die Konzentration des verwendeten organischen Lösungsmittels in Abhängigkeit vom Elutionsvolumen steigt, und zwar vorzugsweise in linearer Abhängigkeit.

Die Einengung des Insulins nach der Chromatographie aus den Eluaten geschieht durch Ausfällung mit Zink oder durch Kristallisation. Dabei kann die Lösung wahlweise vorher mittels Vakuumdestillation weitgehend vom Lösungsmittel befreit oder dessen Konzentration durch Verdünnen mit Wasser reduziert werden. Auf jeden Fall sollte die Lösungsmittelkonzentration vor der Fällung oder Kristallisation bei 10 % oder darunter liegen, um den Proteingehalt im Überstand auf < 50 mg/l zu halten. Die entstandenen Isulinniederschläge lassen sich durch Dekantieren, Zentrifugieren oder Filtration isolieren und trocknen.

Für den zweiten Chromatographieschritt (Verfahrensschritte 2A bis 2B) beträgt die Konzentration der mit Wasser mischbaren organischen Lösungsmittel 1 bis 90 %, bevorzugt 10 bis 60 %, besonders bevorzugt 10 bis 35 %. Die Konzentration der Puffersubstanz beträgt etwa 1 mMol/l bis 2 Mol/l, bezogen auf Wasser als Lösungsmittel, bevorzugt 25 mMol/l bis 1 Mol/l. Die Konzentration der Zwitterionen kann in einem weiten Bereich schwanken. Vorteilhafte Mengen sind 10 mMol/l bis 1 Mol/l, bezogen auf Wasser als Lösungsmittel, bevorzugt 20 mMol/l bis 500 mMol/l.

Die Temperatur während der Chromatographie beträgt 0 °C bis 50 °C, vorzugsweise 15 bis 30 °C, besonders bevorzugt 15 bis 20 °C. Der Betriebsdruck während der Chromatographie ist weitgehend konstant. Die Chromatographie kann mit unterschiedlichem Druck durchgeführt werden, z. B. kann die Chromatographie bei einem Druck von 5 bis 400 bar durchgeführt werden, insbesondere bei 20 bis 100 bar.

Die Beladung der Säulen, Chromatographie und Elution der Insuline und Insulinderivate erfolgt nach bekannten, üblichen, technischen Methoden. Die Beladung der Säule mit den zu reinigenden Insulinen und/oder Insulinderivaten erfolgt mit einem Auflösepuffer, der Aceton oder Acetonitril enthält. Geeignete Puffersubstanzen sind in der Literatur bekannt, beispielsweise Phosphate, Alkali- oder Erdalkalimetallsalze wie Natriumcitrat oder Kaliumacetat, Ammoniumcitrat, Ammoniumacetat, -sulfat oder -chlorid. Ferner können im Auflösepuffer auch Zwitterionen anwesend sein. Die Konzentration des Acetons oder Acetonitrils kann in weiten Grenzen schwanken. Als günstig haben sich Acetonmengen oder Acetonitrilmengen von 5 Volumenprozent bis 50 Volumenprozent erwiesen. Bevorzugt sind Acetonitril- oder Acetonkonzentrationen von 10 bis 40 Volumenprozent, insbesondere von 25 bis 35 Volumenprozent.

Der pH-Wert des Auflösepuffers beträgt etwa pH 1 bis 6, bevorzugt pH 2 bis 5, insbesondere pH 3 bis 4.

Die Säule wird ein- bis fünfmal mit dem Auflösepuffer gewaschen, bevorzugt ein- bis dreimal gewaschen, insbesondere einmal gewaschen. Die Insulinlösung hat einen Proteinanteil von etwa 1 bis 10 %, bevorzugt 3 %.

Die Elution der Insuline erfolgt mit dem erfindungsgemäßen Verfahren bei konstanter Konzentration der Puffersubstanzen und konstanter Konzentration der mit Wasser mischbaren organischen Lösungsmittel (isokratisch) oder durch Veränderung des mit Wasser mischbaren organischen Lösungsmittelanteils im Puffer. Die Veränderung des organischen Lösungsmittelanteils erfolgt in der Weise, daß die Konzentration des verwendeten organischen Lösungsmittels in Abhängigkeit vom Elutionsvolumen steigt, und zwar vorzugsweise in linearer Abhängigkeit.

Die Abtrennung des Insulins nach der Chromatographie aus den Eluaten geschieht durch Ausfällung mit Zink oder durch Kristallisation. Dabei kann die Lösung wahlweise vorher mittels Vakuumdestillation weitgehend vom Lösungsmittel befreit oder dessen Konzentration durch Verdünnen mit Wasser reduziert werden. Auf jeden Fall sollte die Lösungsmittelkonzentration vor der Fällung oder Kristallisation bei 10 % oder darunter liegen, damit der Proteingehalt im Überstand auf weniger als 50 mg/l gehalten wird. Die entstandenen reinen Insulinniederschläge lassen sich durch Dekantieren, Zentrifugieren oder Filtration isolieren und trocknen.

Das erfindungsgemäße Verfahren eignet sich nicht nur zur analytischen Chromatographie, sondern auch zur präparativen Chromatographie, insbesondere wenn das erfindungsgemäße Verfahren mit einer präparativen HPLC-Anlage durchgeführt wird.

Unter dem Begriff "präparative Chromatographie" wird ein Reinigungsverfahren verstanden mit dem Ziel, Reinprodukte zu gewinnen und nicht nur zu analysieren. Die Menge der Reinprodukte kann in weiten Grenzen schwanken, beispielsweise von 1 mg bis 50 kg, bevorzugt von 50 mg bis 15 kg.

In den folgenden Beispielen wird das erfindungsgemäße Verfahren im einzelnen beschrieben. Prozentangaben beziehen sich auf das Gewicht, sofern nicht anders angegeben.

### Beispiel 1

- Puffer A:: 0,1 M Ammoniumsulfat, 0,1 M Glycin, 0,025 M Natriumacetat, pH 5,5, 5 % Propanol
- Puffer B:: 0,1 M Ammoniumsulfat, 0,1 M Glycin, 0,025 M Natriumacetat, pH 5,5, Wasser/n-Propanol im Verhältnis 1:1
- Sorbens: ®Kromasil C8, 13 µm, 100 Å Porenweite
- Säulenabmessung:: 2 cm x 25 cm.

Die Säule wird mit einer Lösung von 0,5 g rohem Humaninsulin aus der gentechnischen Herstellung mit einem Gehalt von 2,5 % acetyliertem Human-Insulin in 25 ml eines Auflösepuffers beladen, der 0,1 M Glycin, 0,1 M NaCl, 32 Vol. -% Aceton, pH 3,5 enthält.

Nach dem Auftragen der Humaninsulinlösung auf die Säule wird mit einem Säulenvolumen des Lösepuffers nachgewaschen.

Innerhalb von 90 min werden bei einem Fluß von 9 ml/min und Erhöhung der Propanolkonzentration von 14 % auf 25 % die einzelnen Proteinkomponenten getrennt eluiert. Man erhält nach Kristallisation bzw. Fällung und Trocknung als Hauptfraktion Humaninsulin mit einer Reinheit von mehr als 98% mit einer Ausbeute von 86,2 %, bezogen auf das eingesetzte Insulin.

Die Bestimmung der Konzentration an A9-acetyliertem Humaninsulin wird wie folgt durchgeführt:

Für die Analyse werden HPLC-Säulen (4 mm x 250 mm) von Fa. Machery & Nagel, Düren, gefüllt mit ®Nucleosil C18, 120 Å, 5 µm eingesetzt.
- Puffer A:: 35 mM Natriumphosphat
300 mM Natriumchlorid
25 % Acetonitril
75 % Wasser pH 3,0
- Puffer B:: 35 mM Natriumphosphat
50 mM Natriumchlorid
65 % Acetonitril
35 % Wasser pH 3,0

Bei einer Säulentemperatur von 40 °C, einem Fluß von 1 ml/min und einem Gradienten von 12 % Puffer 10 auf 21 % Puffer B innerhalb 25 min wird das acetylierte Derivat mit einer Retentionszeitdifferenz von 6 min nach dem Humaninsulinpeak von der Säule eluiert. Mit dieser Analysenmethode läßt sich das Insulinderivat sehr gut von Humaninsulin abtrennen, identifizieren und quantifizieren.

Der Gehalt an A9-acetyliertem Humaninsulin beträgt nach der Reinigung weniger als 0,1 %.

### Beispiel 2

In dem folgenden Vergleichsversuch erfolgt die Chromatographie wie im Beispiel 1, wobei jedoch der Auflösepuffer nur 0,1 M Glycin/HCl, pH 2,8 enthält.
Ausbeute: 58 % mit einer Reinheit von mehr als 98 %.
Der Gehalt an A9-acetyliertem Humaninsulin beträgt 0,2 %.

### Beispiel 3

- Puffer A:: 0,15 M Ammoniumsulfat, 0,15 M Glycin, 0,025 M Natriumacetat, pH 5,5, 5 % n-Propanol;
- Puffer B:: 0,15 M Ammoniumsulfat, 0,15 M Glycin, 0,025 M Natriumacetat, pH 5,5 Wasser/n-Propanol im Verhältnis 1 : 1.
- Sorbens:: Kromasil C8, 13 µm, 100 Å Porenweite, Fa. EKA Nobel.
- Säulenabmessung:: 6 cm x 25 cm.

Die Säule wird mit einer Lösung von 10 g Reaktionsgemisch aus der Umamidierung von Schweineinsulin mit Trypsin, gelöst in 200 ml 0,1 M Glycin/HCl-Puffer, pH 2,8, beladen. Die Trypsinaktivität beträgt mehr als 10000 mAU/min. Innerhalb von 120 Minuten werden bei einem Fluß von 40 ml/min und Erhöhung der Propanolkonzentration von 14 auf 30 % die einzelnen Proteinkomponenten getrennt eluiert. Man erhält nach Kristallisation bzw. Fällung und Trocknung als Hauptfraktion Humaninsulin-B30-di-tert.butylthreoninester/ether mit einer Reinheit von > 97 % mit einer Ausbeute von 93 %, bezogen auf das eingesetzte Insulin.

Die Bestimmung der Proteaseaktivität wird wie folgt durchgeführt:

Bei diesem Test wird die Kinetik der Abspaltung eines Chromophors (4-Nitroanilid) von dem Substrat Carbobenzoxy-Val-Gly-Arg-4-nitroanilid-acetat (Chromozym-Try; Best.-Nr. 378488 Fa. Boehringer Mannheim) bestimmt. Der Fortgang der Abspaltungsreaktion wird spektralphotometrisch über einen Zeitraum von 60 min. bei einer Wellenlänge von 405 nm gemessen. Die Zunahme der gemessenen Absorption ist dabei direkt proportional zur tryptischen Aktivität in dem Produkt. Die Steigung der Absorptionsgeraden wird bestimmt (Einheit: milli absorption units pro Minute (mAU/min.)) und ist ein direktes Maß für den Proteasegehalt.

Der Test wird wie folgt durchgeführt:

7,5 mg Humaninsulin werden in einem Milliliter Lösepuffer unter Rühren innerhalb von 30 min gelöst.
- Lösepuffer:: 200 mM TRIS pH 8,0 1 mM EDTA

Das Chromozymsubstrat wird mit einer Konzentration von 1 mg/ml in Wasser gelöst.

Die Reaktion wird bei 37 °C durch Zugabe von 200 µl Substratlösung zu 1 ml Probelösung gestartet und dann sofort spektralphotometrisch bei 405 nm vermessen. Die Absorption der Reaktionslösung bei dieser Wellenlänge wird kontinuierlich für 60 Minuten registriert. Zur Ermittlung der tryptischen Aktivität wird die Steigung der Absorptionsgeraden bestimmt.

Es wird eine tryptische Aktivität von 250 mAU/min gemessen.

### Beispiel 4

Die Chromatographie erfolgt wie in Beispiel 3 beschrieben, wobei die Bedingungen wie folgt geändert wurden:
0,1 M Glycin
0,05 M Ammoniumsulfat
Säulenabmessungen: 6 cm x 25 cm

Es wird eine tryptische Aktivität von 5 bis 10 mAU/min gemessen.

### Beispiel 5

Die Chromatographie erfolgt wie in Beispiel 4 beschrieben, wobei die Säulenabmessungen 30 cm x 30 cm betragen.

Es wird eine tryptische Aktivität von 20 bis 25 mAU/min gemessen.

### Beispiel 6

- Puffer A:: 0,05 M Ammoniumsulfat, 0,1 M Glycin, 0,025 M Natriumacetat, pH 5,5; 5 % Propanol;
- Puffer B:: 0,05 M Ammoniumsulfat, 0,1 M Glycin, 0,025 M Natriumacetat, pH 5,5, Wasser/n-Propanol im Verhältnis 1 : 1.
- Sorbens:: Kromasil C8, 13 µm, 100 Å Porenweite, Fa. EKA Nobel.
- Säulenabmessung:: 6 cm x 25 cm.

Die Säule wird mit einer Lösung von 10 g Reaktionsgemisch aus der Trifluoressigsäurespaltung von Humaninsulin-B30-di-tert.butylthreoninester/ether, mit einem Proteinanteil von 79 % beladen und wie in Beispiel 4 beschrieben chromatographiert. Anschließend wird wie in Beispiel 3 gefällt um die insulinhaltigen Fraktionen einzuengen und in einen Gemisch aus 200 ml 0,1 M Glycin, 0,1 M NaCl, 32 Volumenprozent Aceton, pH 3,5, gelöst und die Säule damit beladen. Nach dem Auftragen der Humaninsulinlösung auf die Säule wird mit einem Säulenvolumen des Auflösepuffers nachgewaschen. Anschließend erfolgt die Elution durch einen Gradienten von 17 % Puffer B auf 19 % B innerhalb von 45 min.

Alle anderen Bedingungen sind analog Beispiel 4.

Die tryptische Aktivität beträgt weniger als 0,05 mAU/min.

### Beispiel 7

Die Chromatographie erfolgt wie in Beispiel 6. Die Säulenabmessungen sind 30 cm x 30 cm. Die tryptische Aktivität beträgt weniger als 0,05 mAU/min.

Sequenzprotokoll

SEQ ID NO: 1
Art der Sequenz: Aminosäure (AS)
Sequenzlänge: 20 AS

Gly Ile Val Glu Gln Cys Cys Thr Ser Ile Cys Ser Leu
1               5                   10

Tyr Gln Leu Glu Asn Tyr Cys
    15                  20

SEQ ID NO: 2
Art der Sequenz: AS
Sequenzlänge: 29 AS

Phe Val Asn Gln His Leu Cys Gly Ser His Leu Val Glu
1               5                   10

Ala Leu Tyr Leu Val Cys Gly Glu Arg Gly Phe Phe Tyr
    15                  20                  25

Thr Pro Lys

## Patentansprüche

1. Verfahren zur Gewinnung von Insulin, das annähernd frei ist von Proteasen und/oder an Position A9-acetyliertem Insulin durch Chromatographie in wäßrigen, gepufferten Elutionsmitteln, die mit Wasser mischbare, organische Lösungsmittel enthalten, an lipophil modifiziertem Kieselgel, dadurch gekennzeichnet, daß man
1A) das Gemisch aus Proteasen und/oder an Position A9-acetyliertem Insulin und Insulin in einem Puffer löst,
1B) die Elution mit einem gepufferten Elutionsmittel, das Zwitterionen enthält und gegebenenfalls auf einen pH-Wert in der Nähe des isoelektrischen Punktes des zu reinigenden Insulins eingestellt worden ist, durchführt,
die gewonnenen Insulinfraktionen gegebenenfalls einengt und anschließend die gewonnenen Insulinfraktionen
2A) in einem Auflösepuffer, der Aceton oder Acetonitril enthält, löst,
2B) die erhaltene Lösung auf die Säule aufträgt,
2C) die Säule mit dem Auflösepuffer nachspült und
2D) das zu reinigende Insulin mit einem gepufferten Elutionsmittel, das Zwitterionen enthält und gegebenenfalls auf einen pH-Wert in der Nähe des isoelektrischen Punktes des zu reinigenden Insulins eingestellt worden ist, eluiert.

2. Verfahren nach Anspruch 1, dadurch gekennzeichnet, daß die Verfahrensschritte 2A bis 2D angewendet werden zur Gewinnung von Insulin, das annähernd frei ist von an Position A9-acetyliertem Insulin aus Gemischen enthaltend Insulin, Proteasen und an Position A9-acetyliertes Insulin, oder Insulin und an Position A9-acetyliertes Insulin.

3. Verfahren nach Anspruch 1 oder 2, dadurch gekennzeichnet, daß der Auflösepuffer 5 bis 50 Volumenprozent, insbesondere 10 bis 40 Volumenprozent Aceton enthält.

4. Verfahren nach einem oder mehreren der Ansprüche 1 bis 3, dadurch gekennzeichnet, daß der Auflösepuffer einen pH-Wert von 2 bis 5, insbesondere 3 bis 4, hat.

5. Verfahren nach einem oder mehreren der Ansprüche 1 bis 4, dadurch gekennzeichnet, daß ein Insulin der Formel I, 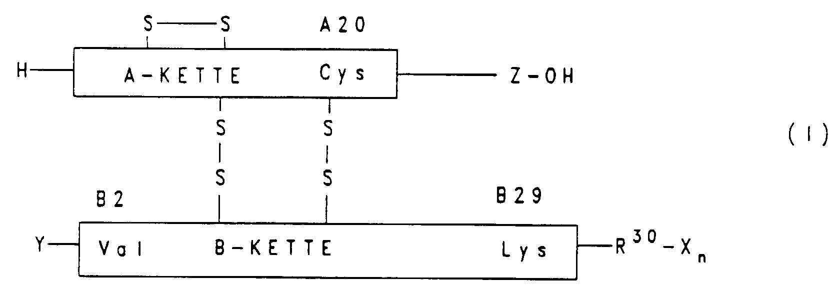 in welcher
R³⁰ für den Rest einer genetisch kodierbaren L-Aminosäure,
X für eine Hydroxygruppe, eine physiologisch unbedenkliche organische Gruppe basischen Charakters mit bis zu 50 C-Atomen, eine genetisch kodierbare L-Aminosäure und deren gegebenenfalls vorhandene endständige Carboxylgruppe frei, als Esterfunktion, als Amidfunktion, als Lacton oder zu CH₂OH reduziert vorliegen kann,
n für eine ganze Zahl von 0 bis 10
Y für Wasserstoff oder L-Phenylalanin und
Z für eine genetisch kodierbare L-Aminosäure steht,
und die A- und B-Kette die Sequenzen tierischen oder menschlichen Insulins haben, gewonnen wird.

6. Verfahren nach Anspruch 5, dadurch gekennzeichnet, daß ein Insulinderivat der Formel I, wobei R³⁰ für L-Alanin oder L-Threonin steht und X für eine oder mehrere L-Aminosäuren aus der Gruppe L-Arginin, L-Lysin oder L-Phenylalanin, Z für L-Glycin, L-Alanin, L-Serin, L-Threonin, L-Asparaginsäure oder L-Glutaminsäure steht und A1 bis A20 oder B2 bis B29 für die Aminosäuresequenz von Human-, Schweine- oder Rinderinsulin steht, gewonnen wird.

7. Verfahren nach einem oder mehreren der Ansprüche 1 bis 6, dadurch gekennzeichnet, daß Humaninsulin gewonnen wird.

8. Verfahren nach einem oder mehreren der Ansprüche 1 bis 7, dadurch gekennzeichnet, daß als Protease Trypsin abgetrennt wird.

9. Verfahren nach einem oder mehreren der Ansprüche 1 bis 8, dadurch gekennzeichnet, daß eine präparative Hochdruckflüssigkeitschromatographie-Anlage eingesetzt wird.

## Claims

1. A process for obtaining insulin, which is virtually free from proteases and/or insulin acetylated at position A9, by chromatography in aqueous, buffered eluents, which contain water-miscible organic solvents, on lipophilically modified silica gel, wherein
1A) the mixture of proteases and/or insulin acetylated at position A9 and insulin is dissolved in a buffer,
1B) the elution is carried out with a buffered eluent which contains zwitterions and optionally has been adjusted to a pH in the vicinity of the isoelectric joint of the insulin to be purified,
the insulin fractions obtained are concentrated as required and subsequently the insulin fractions obtained
2A) are dissolved in a dissolving buffer which contains acetone or acetonitrile,
2E) the solution obtained is added to the column,
2C) the column is washed with the dissolving buffer and
2D) the insulin to be purified is eluted with a buffered eluent which contains zwitterions and optionally has been adjusted to a pH in the vicinity of the isoelectric point of the insulin to be purified.

2. The process as claimed in claim 1, wherein the process steps 2A to 2D are used for obtaining insulin, which is virtually free from insulin acetylated at position A9, from mixtures containing insulin, proteases and insulin acetylated at position A9, or insulin and insulin acetylated at position A9.

3. The process as claimed in claims 1 or 2, wherein the dissolving buffer contains 5 to 50 percent by volume, in particular 10 to 40 percent by volume of acetone.

4. The process as claimed in one or more of claims 1 to 3, wherein the dissolving buffer has a pH of 2 to 5, in particular 3 to 4.

5. The process as claimed in one or more of claims 1 to 4, wherein an insulin is obtained of the formula I, 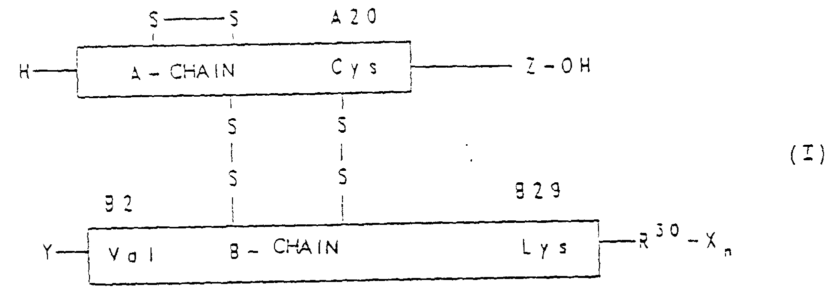 in which
R³⁰ is the residue of a genetically encodable L-amino acid,
x is a hydroxyl group, a physiologically acceptable organic group of basic character with up to 50 carbon atoms, a genetically encodable L-amino acid whose terminal carboxyl group, if present, can exist free, as an ester function, as an amide function, as a lactone or reduced to CH₂OH,
n is an integer from 0 to 10,
Y is hydrogen or L-phenylalanine, and
Z is a genetically encodable L-amino acid,
and the A and B chains have the sequences of animal or human insulin.

6. The process as claimed in claim 5, wherein an insulin derivative of the formula I is obtained, in which R³⁰ is L-alanine or L-threonine and X is one or more L-amino acids from the group comprising L-arginine, L-lysine or L-phenylalanine, Z is L-glycine, L-alanine, L-serine, L-threonine, L-aspartic acid or L-glutamic acid and A1 to A20 or B2 to B29 is the amino acid sequence of human insulin, porcine insulin or bovine insulin.

7. The process as claimed in one or more of claims 1 to 6, wherein human insulin is obtained.

8. The process as claimed in one or more of claims 1 to 7, wherein the protease which is separated off is trypsin.

9. The process as claimed in one or more of claims 1 to 8, wherein preparative high pressure liquid chromatography equipment is employed.

## Revendications

1. Procédé pour l'obtention d'insuline qui est pratiquement exempte de protéase et/ou d'insuline acétylée en position A9 par chromatographie dans des milieux d'élution tamponnés aqueux, qui contiennent des solvants organiques miscibles à l'eau, sur gel de silice modifié de manière lipophile, caractérisé en ce que l'on
1A) dissout le mélange de protéases et/ou l'insuline acétylée en position A9 et l'insuline dans un tampon,
1B) effectue l'élution avec un milieu d'élution tamponné qui contient des zwitterions et on l'ajuste éventuellement à une valeur de pH proche du point isoélectrique de l'insuline à purifier
concentre éventuellement les fractions d'insuline obtenues et en ce qu'ensuite
2A) on dissout les fractions d'insuline obtenues dans un tampon de mise en solution, qui contient de l'acétone ou de l'acétonitrile,
2B) on fait passer la solution obtenue sur la colonne,
2C) on rince la colonne avec le tampon de mise en solution et
2D) on élue l'insuline à purifier avec un milieu d'élution tamponné qui contient des zwitterions et on l'ajuste éventuellement à une valeur de pH proche du point isoélectrique de l'insuline à purifier.

2. Procédé selon la revendication 1, caractérisé en ce que l'on utilise les étapes de procédé 2A à 2D pour l'obtention d'insuline, qui est pratiquement exempte d'insuline acétylée en position A9, à partir de mélanges contenant de l'insuline, des protéases, et de l'insuline acétylée en position A9, ou d'insuline et d'insuline acétylée en position A9.

3. Procédé selon la revendication 1, ou la revendication 2 caractérisé en ce que le tampon de mise en solution contient 5 à 50% en volume, de préférence 10 à 40% en volume d'acétone.

4. Procédé selon une ou plusieurs des revendications 1 à 3, caractérisé en ce que le tampon de mise en solution a un pH de valeur comprise entre 2 et 5, de préférence entrée 3 et 4.

5. Procédé selon une ou plusieurs des revendications 1 à 3, caractérisé en ce que l'on obtient une insuline de Formule I 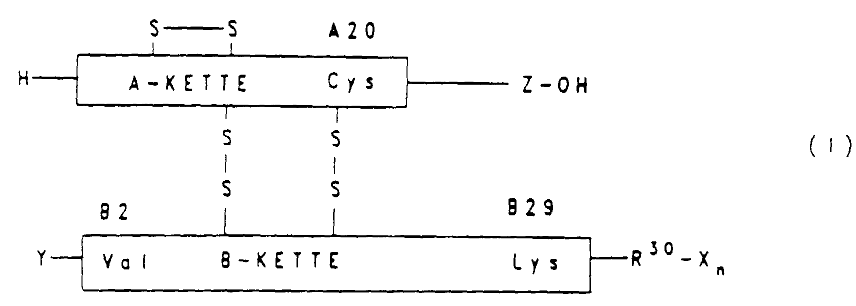 dans laquelle
R³⁰ représente le résidu d'un L-aminoacide génétiquement codant,
x représente un groupe hydroxy, un groupe organique de caractère basique physiologiquement acceptable ayant jusqu'à 50 atomes de carbone, un L-aminoacide génétiquement codant dont le groupe carboxylique existant en bout de chaîne peut se présenter sous forme libre, sous forme de fonction ester, fonction amide, de lactone ou sous la forme réduite CH₂OH,
n représente un nombre entier compris entre 0 et 10,
Y représente un atome d'hydrogène ou la L-phénylalanine et
Z représente un L-aminoacide génétiquement codant
et les chaînes A et B ont les séquences de l'insuline animale ou humaine.

6. Procédé selon la revendication 5, caractérisé en ce que l'on obtient un dérivé de l'insuline de Formule I dans laquelle R³⁰ représente la L-alanine ou la L-thréonine et X représente un ou plusieurs L-aminoacides appartenant au groupe de la L-arginine, la L-lysine, ou la L-phénylalanine, Z représente la L-glycine, la L-alanine, la L-sérine, la L-thréonine, l'acide L-aspartique ou l'acide L-glutamique, et A1 à A20 ou B2 à B29 représente la séquence d'aminoacides de l'insuline humaine, de porc ou de boeuf.

7. Procédé selon une ou plusieurs des revendications 1 à 6, caractérisé en ce que l'on obtient de l'insuline humaine.

8. Procédé selon une ou plusieurs des revendications 1 à 7, caractérisé en ce que la trypsine est séparée en tant que protéase.

9. Procédé selon une ou plusieurs des revendications 1 à 8, caractérisé en ce que l'on utilise un dispositif de chromatographie préparative liquide à haute pression.
